# EUROPÄISCHE PATENTSCHRIFT

(11) **EP 1 509 175 B1**
(45) Veröffentlichungstag und Bekanntmachung des Hinweises auf die Patenterteilung: **02.01.2008**
(21) Anmeldenummer: 03735447.9
(22) Anmeldetag: 23.05.2003
(51) Int. Cl.: A61F 2/44, A61F 2/46

(54) **DILATIERBARES BALLONIMPLANTAT**
DILATABLE BALLOON IMPLANT
IMPLANT DE TYPE BALLON GONFLABLE

(30) Priorität: 25.05.2002 DE 10223332
(43) Veröffentlichungstag der Anmeldung: 02.03.2005
(73) Patentinhaber: EFMT Entwicklungs- und Forschungszentrum Für Mikrotherapie GmbH, 44799 Bochum (DE)
(72) Erfinder: GRÖNEMEYER, Dietrich, 45549 Sprockhövel (DE); DELI, Martin, 45472 Mülheim (DE); SPEDER, Jürgen, 44807 Bochum (DE); RICHTER, Jörn, 79400 Kandern (DE)
(74) Vertreter: Thiel, Christian
(86) Internationale Anmeldenummer: PCT/EP2003/005407
(87) Internationale Veröffentlichungsnummer: WO 2003/099171

(56) Entgegenhaltungen:
- WO-A-00/67650
- WO-A-01/28464
- WO-A-99/02108
- WO-A-99/02214
- WO-A-03/007853
- US-A- 5 571 189
- US-A1- 2002 010 472

## Beschreibung

Die Erfindung betrifft ein dilatierbares Ballonimplantat. Darüber hinaus betrifft die Erfindung eine Vorrichtung zur Deponierung eines solchen Implantates in Knochenhohlräumen.

Zur Behandlung frakturierker Wirbelkörper (insbesondere infolge osteoporotischer Knochengewebsveränderungen) ist es bekannt, direkt Knochenzement oder ein anderes Füllmaterial durch einen perkutanen Zugang in den Wirbelkörper zu füllen und diesen so zu stabilisieren (Vertebroplastie). Nachteilig bei dieser Verfahrensweise ist, daß der Wirbel vor Stabilisierung nicht aufgerichtet werden kann. Überdies besteht bei diesem Verfahren die Gefahr, daß Füllmaterial aus der inneren Wirbelcavität heraustritt und in den Körper gelangt.

Darüber hinaus kann auch Material in den Spinalkanal, das Neuroforamen oder den venösen Plexus des Wirbelkörpers gelangen und diesen verschließen oder dadurch über den Blutstrom verteilt werden und so zu Embolien und Infarkten führen. Besonders problematisch ist hierbei, daß bei zu geringer Viskosität des Füllmaterials die Gefahr des Materialaustritts aus der Wirbelcavität steigt, andererseits bei zu hoher Viskosität das Material zu früh aushärtet, was seinerseits eine unzureichende Füllung nach sich zieht.

Gemäß eines weiteren Verfahrens des Standes der Technik wird die Spongiosa des Wirbels vor der Verfüllung der Cavität mit Füllmaterial mit einem oder mehreren Ballonkathetem komprimiert und so aufgeweitet (Kyphoplastie). Dies soll einerseits zu einer Abdichtung von Rissen führen und somit einem möglichen Austritt von Füllmaterial entgegenwirken, andererseits auch der möglichen Aufrichtung des Wirbelkörpers vor Verfüllung dienen. Die bei diesem Verfahren notwendige Entfernung des Ballonkatheters verlängert die Eingriffszeit und birgt die Gefahr, daß der Tonus der Rückenmuskulatur eine zuvor erreichte Aufrichtung wieder rückgängig macht. Auch kann durch die Kyphoplastie der Austritt von Füllmaterial in den Spinalkanal, in ein Neuroforamen oder den venösen Plexus nicht gänzlich verhindert werden.

Angesichts der mit dem Stand der Technik verbundenen Probleme bei der Behandlung frakturierter Wirbelkörper besteht die Aufgabe der Erfindung in der Bereitstellung eines Implantats, das das Risiko des Auslaufens von Füllmaterial bei der Vertebroplastie bei möglichst kurzer Eingriffszeit minimiert und zur Aufrichtung des Wirbelkörpers geeignet ist.

Aus US-A-5,571,189 ist ein dilatierbares Ballonimplantat aus einem flüssigkeitsdurchlässigen Gewebe bekannt, das zur Implantierung in Wirbelkörper bestimmt ist und so ausgebildet ist, das festes Füllmaterial nicht wieder austreten kann. Auch kleine Partikel des eingebrachten Materials, wie beispielsweise Knochenspan oder andere Feststoffteilchen aus knochenbildendem Material, sollen zuverlässig im Ballonimplantat zurückgehalten werden.

US 2002/010472 A1 beschreibt ein gleichartiges Ballonimplantat.

Entsprechend betrifft die Erfindung ein Ballonimplantat mit den Merkmalen des Anspruchs 1.

Das erfindungsgemäße Ballonimplantat wird durch perkutanen Zugang im delatierten Zustand mit einer röhrenförmigen Einführhülse in den vorbereiteten (also eröffneten) Wirbelkörper eingeführt, wo es vorzugsweise durch Einstrom von Füllmaterial durch die Einführhülse dilatiert wird. Die begrenzte Flüssigkeitsdurchlässigkeit gewährleistet einerseits, daß die Gefahr eines Materialaustritts in den Spinalkanal minimiert wird, und ermöglicht andererseits die sichere Verbindung des Implantats mit dem Knochen. Auf diese Weise kann Füllmaterial mit geringerer Viskosität eingesetzt werden, als im Stand der Technik üblich, so daß auch die Gefahr des Aushärtens vor vollständiger Verfügung der Wirbelcavität ausgeschaltet ist. Überdies wird durch den infolge des dilatierenden Ballons ausgeübten Druck ein Aufrichten des Wirbelkörpers ermöglicht, falls erwünscht.

Nach Verfüllung der Cavität wird die proximale Einströmöffnung des Implantats verschlossen und das Implantat von der Einführhülse abgelöst.

Der Einsatz des erfindungsgemäßen Implantats führt zu einem gegenüber Operationsmothoden des Standes der Technik verbesserten Operationsergebnis, indem es die mit dem übermäßigem Austritt von Füllmaterial und überlange Operationsdauern verbundenen Komplikationen minimiert.

In der einfachsten Ausführungsform besteht es nur aus dem Ballon selbst. Es kann darüber hinaus jedoch mit weiteren Mitteln ausgerüstet sein (z. B. Verbindungsmitteln). In diesem Fall ist zweckmäßigerweise nur der Ballon selbst begrenzt flüsslgkeitsdurchlässig

Der Ballon besteht dabei aus einem flüssigkeitsundurchlässigen Material, welches mit Poren zum begrenzten Flüssigkeitsdurchlass versehen ist.

Die Materialien können elastisch oder nicht elastisch ausgebildet sein, so lange sie die Anforderungen an medizinische Materialien hinsichtlich physiologischer Verträglichkeit und Reißfestigkeit erfüllen. Gemäß einer bevorzugten Ausführungsform werden elastische Materialien verwendet, da diese bei der Einführung in die Cavität besonders klein gehalten sein können und so beispielsweise mit Kathetern von besonders geringem Durchmesser zu Einsatz kommen können.

Als flüssigkeitsundurchlässiges, mit Poren versehenes Material eignet sich insbesondere perforiertes oder punktiertes Latex, da dieses im allgemeinen gut verträglich und elastisch ist.

Die Porengröße, und -anzahl (d. h. die Porendichte) des an sich flüssigkeitsundurchlässigen Materials hängt von dem verwendeten Füllmaterial (z. B. herkömmlicher Knochenzement oder polymerisierende Füllmaterialien) ab und ist vom zuständigen Fachmann einfach für das jeweilige Füllmaterial zu bestimmen. Für gängige Materialien eignet sich insbesondere eine Porendichte von bis zu 5 %, vorzugsweise 1 bis 3 %, bezogen auf die Fläche des Materials.

Die Poren sind so zu bemessen, daß In der Therapie Knochenfüllmaterial in einer Menge von 0,05 bis 6, vorzugsweise von 1 bis 4 und besonders bevorzugt 2 bis 3 % austritt und die Verankerung des Implantates durch eine große Porenanzahl sichergestellt ist.

Zur Ausbildung des Ballons sind Materialien (an sich wasserdurchlässig bzw. an sich nicht wasserdurchlässig aber mit Poren versehen) besonders zweckmäßig, die eine Durchlässigkeit des Ballons für medizinisches Knochenfüllmaterial von 0,05 bis 6, vorzugsweise von 1 bis 4 und besonders bevorzugt 2 bis 3 % gewährleisten.

Diese Durchlässigkeit bezeichnet den Anteil des ausgetretenen, im flüssigen Zustand eingebrachten Füllmaterials, bis zu seinem Aushärten in der Cavität. Die insgesamt in den Ballon eingebrachte Menge an Füllmaterial stellt dabei 100 % dar. Das vom Fachmann einzusetzende Material hängt somit vom zu verwendenden Füllmaterial und dessen Viskosität ab. Der Fachmann kann das für das jeweils zu verwendende Füllmaterial bestgeeignete Ballonmaterial einfach bestimmen (niedrigere Viskosität bzw. langsameres Aushärten - höhere Materialdichte).

Das erfindungsgemäße Implantat erlaubt dabei den Einsatz besonders niedrig viskoser Füllmaterialien (wobei ein dichteres Material einzusetzen ist, so daß zweckmäßigerweise eine Durchlässigkeit wie vorstehend erläutert erzielt wird), was das Risiko des Aushärtens vor Operationsbeendigung wesentlich reduziert und überdies den Einsatz von Einführhülsen und anderer Gerätschaften geringerer Durchmesser erlaubt. Eine solch niedrige Durchlässigkeit minimiert das mit zu großem Austritt von Füllmaterial verbundene Operationsrisiko und ermöglicht dennoch eine Fixierung des Implantats in der Wirbelcavität.

Im nicht dilatierten Zustand nimmt der Ballon eine sackartige Form ein. Gemäß einer zweckmäßigen Ausführungsform nimmt er im dilatierten Zustand ohne Einwirkung äußerer Zwänge (beispielsweise durch die Form der Wirbelcavität) eine vorbestimmte Form ein, z. B. eine im wesentlichen kugelförmige, quaderförmige oder auch komplexere Form. Auf diese Weise kann für jede Ausbildung einer Wirbelcavität ein besonders gut angepaßtes Implantat eingesetzt werden, welches die vollständige Verfüllung besonders sicher gewährleistet. Diese Sicherheit wird erhöht durch den Einsatz elastischer Materialien für den Ballon, welche die besonders gute Anpassung der dilatierten Ballonform an die der Cavität gewährleisten.

Gemäß einer anderen zweckmäßigen Ausführungsform weist das Implantat zur Abtrennung vom Einführmittel am proximalen, offenen Ende eine Materialschwächung als Sollbruchstelle auf. Diese besonders kostengünstige Variante ermöglicht den Ballonverschluß und die Abtrennung des Implantats vom Einführmittel durch Drehen in Verbindung mit leichter Zugausübung nach Verfüllung der Höhle, so daß es sich in Folge seines Zugwiderstandes einfach abtrennen lässt (der Zugang zur Wirbelcavität sollte dabei einen geringeren Durchmesser haben als die Cavität selbst, so daß das Implantat nach Verfüllung darin auch vor Materialaushärtung bereits mechanisch durch Verklemmung gesichert sitzt).

Gemäß einer weiteren zweckmäßigen Ausführungsform weist das Implantat im proximalen Bereich ein Verbindungsmittel zur lösbaren und leitenden Verbindung mit einer Einführhülse auf. Abhängig von der Art des verwendeten Verbindungsmittels erfolgt eine Entkopplung von der Einführhülse von außen beispielsweise auf mechanischem Weg, durch elektrolytische Ablösung oder Einwirkung von Laserblitzen. Solche Verbindungsmittel und die dazu nötigen Materialien sind dem zuständigen Fachmann bekannt.

Zweckmäßig ist auch eine Ausführungsform, bei der das Implantat eine Verschlußvorrichtung für die proximale Öffnung aufweist, die durch den Operateur von außen bedienbar ist (z. B. ein Stopfen oder eine durch Zug verengbare Schlinge).

Die Erfindung bezieht sich überdies auf eine Vertebroplastie-Vorrichtung mit einem Ballonimplantat wie vorstehend beschrieben, das mit dem distalen Ende einer Einführhülse verbunden Ist, so daß sein Inneres mit dem Einführhülsenlumen kommuniziert (distales Ende bedeutet dabei nicht notwendigerweise unmittelbares Ende der Einführhülse sondern den distalen Endbereich).

Das Implantat kann mit der Einführhülse dabei grundsätzlich durch alle zweckmäßigen Arten der Befestigung verbunden sein - so ist eine Befestigung am äußeren Umfang oder am inneren Umfang der EInführhülse ebenso denkbar wie ein kontinuierlicher Übergang zur Einführhülse.

Zweckmäßig ist eine Vorrichtung, bei der der Ballon mit der Einführhülse durch formschlüssige Verbindung, Verkleben, Verklemmen, Spreizverbindung oder Verschraubung verbunden ist. Der Ballon und/oder die Einführhülse sind in diesen Ausführungsformen nötigenfalls mit den jeweils entsprechenden Haltemitteln versehen.

Die Abtrennbarkeit des Implantats von der Einführhülse wird dadurch gewährleistet, daß entweder die Verbindung zwischen Einführhülse und Ballon selbst lösbar ist (z. B. oben genannte Verschraubung, Verklemmung oder elektrolytische Lösbarkeit eines oder mehrerer Verbindungsmittel) oder die Verbindung zwischen Einführhülse und Ballon selbst unlösbar ist, wobei der Ballon mit einer Sollbruchstelle (vgl. vorstehend) ausgestattet ist.

Zweckmäßig ist eine Vorrichtung, bei der der Ballon mit seinem offenen Ende im inneren Umfang der Einführhülse befestigt ist.

Gemäß einer anderen besonders zweckmäßigen Ausführungsform ist der Ballon mit seinem offenen Ende über das distale Einführhülsenende gestülpt und mit dem äußeren Einführhülsenumfang verbunden.

Bei einer besonders vorteilhaften Ausgestaltung der Vorrichtung ist die Einführhülse am distalen Ende mit einem. Sicherungsmittel versehen, daß der zusätzlichen Sicherung der Verbindung von distalem Einführhülsenende und proximalem Implantatende dient. Es kann Bestandteil der Einführhülsenwandung selbst sein (umgebogenes distales Ende) oder ein zusätzliches Element (z. B. Ring), weiches mit der Einführhülse auf herkömmliche Weise fest verbunden ist.

Vorzugsweise ist es als Klemm- oder Spannring ausgebildet, welcher als Stopperwust das proximale Ende des Ballons fest mit der Einführhülse verklemmt (also zusätzlich mit der Außenwandung verbindet) oder verspannt (d. h. gegen die Innenwandung drückt).

Die Einführhülse besteht vorzugsweise aus einem medizinischen Stahl. Grundsätzlich eignen sich zur Ausbildung derselben jedoch alle physiologisch verträglichen Materialien hoher Beanspruchbarkeit und Festigkeit.

Gemäß einer besonders bevorzugten Ausführungsform wird die Einführhülse durch die Kanüle eines medizinischen Trokars als Einführhilfe geführt.

Die Erfindung wird im folgenden anhand von in der Figur dargestellten Ausführungsbelspielen näher erläutert.

Es stellen dar:

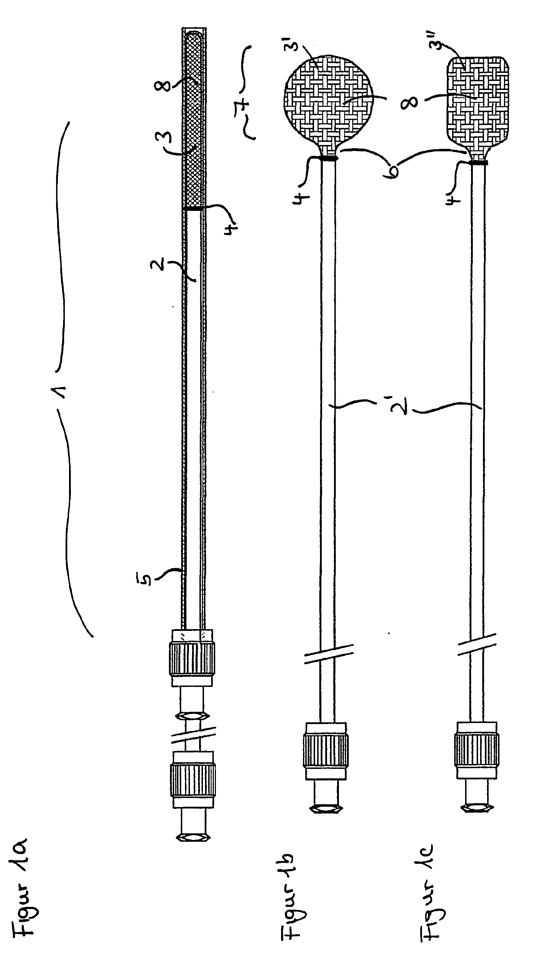

- Figur 1a: in nicht maßstabsgetreuer Widergabe eine Vertebroplastie-Vorrichtung 1 mit Katheter 2 vor Einführung in die Cavität
- Figur 1b: in nicht maßstabsgetreuer Widergabe eine Vertebroplastie-Vorrichtung 1 mit kugelförmigem dilatierten Ballonimplantat 3
- Figur 1c: in nicht maßstabsgetreuer Widergabe eine Vertebroplastie-Vorrichtung 1 mit quaderförmigem dilatierten Ballonimplantat 3

Die in Figur 1a dargestellte Vertebroplastie-Vorrichtung 1 umfaßt eine Einführhülse 2 und ein dilatierbares Ballonimplantat 3. Das Ballonimplantat 3 ist am distalen Ende der Einführhülse 2 durch Heißverformung formschlüssig auf die Außenwandung der Einführhülse 2 aufgebracht. Zur zusätzlichen Sicherung ist sie mittels Verklemmung zwischen einem als Stopperwust 4 ausgebildetem Klemmring und dem Außenumfang der Einführhülse 2 gegen ein Verrutschen nach distal gesichert.

Nach Eröffnen eines perkutanen Zugangs und Eröffnung des Wirbelkörpers mittels bekannter Techniken, etwa eines Trokars, wird die Einführhilfe 5, etwa das Kanülenrohr des Trokars, bis an Öffnung des Wirbels vorgeführt und das Ballonimplantat 3 im deflatierten Zustand in die Wirbelkavität eingebracht.

Dazu wird unter röntgenologischer Kontrolle die Einführhülse 2 zunächst bis an die Wirbelkörperöffnung herangeführt. Danach wird der Ballon durch Vorschieben der Einführhülse 2 in die Cavität eingebracht, wobei zur vollständigen Einführung auch das distale Ende der Einführhülse 2 in die Cavität vorgeschoben wird. Der Eingriff erfolgt unter röntgenologischer Kontrolle, wobei die Positionierung von Einführhilfe oder Katheter 5 und Einführhülse 2 durch die Anordnung von Markern Oberprüfbar ist.

Anschließend wird durch die Einführhülse 2 von außen geeignetes Füllmaterial (Polymethylmethacrylat, Knochenzement oder ein anderes geeignetes Material, vorzugsweise röntgendicht) in das sich in der Cavität befindliche Ballonimplantat 3 eingebracht, bis dieses die Cavität ausfüllt.

In den Figuren 1b und 1c sind zwei verschiedene Ausführungsformen des Implantats 3'/3" dargestellt, welche im dilatierten Zustand unterschiedliche Raumformen einnehmen (Kugel 3', bzw. Quader 3"). Durch diese Ausgestaltung kann abhängig von der Form der Cavität ein Implantat 3 ausgewählt werden, welches bereits von vorneherein eine der Form der Cavität besonders angepasste Gestalt annimmt. Die Elastizität des Nylonmaterials bedingt überdies, daß sich die Implantatform bei Dilatation des Implantats 3 über seine vorbestimmte Form hinaus weiter in die Cavität einpasst und diese so besonders gut ausfüllt.

Das Implantat 3 besteht in diesem Beispiel aus dem Ballon selbst. Dieser ist so vorgeformt, daß er im dilatierten Zustand einen Hals 6 (geringerer Außenumfang im Vergleich zum Körper 7) und einen Körper 7 aufweist. In dem Halsbereich 7 ist das Implantat 3 mit einer Materialschwächung ausgerüstet. Diese bewirkt, daß das Implantat 3 sich nach Füllung mit dem Füllmaterial einfach von außen durch leichte Drehbewegung bei gleichzeitigem leichten Zug an der Einführhülse 2 von dieser abtrennen läßt.

Die Drehbewegung führt, zusammen mit der Elastizität des für das Implantat 3 verwendeten Nylonmaterials 8, gleichzeitig dazu, daß sich der Hals 6 des Implantats 3 verschließt, so daß auch nach proximal keine größeren Mengen Füllmaterial austreten können.

Überdies können geeignete Verschlussmittel für die Cavität (z. B. Stopfen oder Schrauben) eingesetzt werden, welche die Cavität nach Verfüllung verschließen. Auch ist der Einsatz von Implantaten möglich, welche selbst Verschlußmittel aufweisen (nicht dargestellt).

Darüber hinaus kann beispielsweise nach Durchführung einer bis zum Verschluß des Halses 6 führenden Drehbewegung mit der Abtrennung des Implantats 3 (durch Zugeinwirkung und weitere Verdrehung) abgewartet werden, bis das Füllmaterial ausgehärtet ist. Dabei muss sichergestellt sein, daß das distale Ende der Einführhülse 2 vor Aushärten aus dem Wirbel entfernt und an der Öffnung der Cavität positioniert wird. Auf diese Weise wird die versehentliche Verbindung der Einführhülse 2 mit dem Wirbel vermieden.

Die Einführhilfe 5 ist in diesen Ausführungsformen als Kanüle eines medizinischen Trokars ausgebildet. Die Einführhülse 2 mit dem Implantat 3 wird in der Einführhilfe 5 zum Einsatzort vorgeschoben.

Zunächst wird das Ensemble aus Einführhilfe und Obturator (Trokar) unter Röntgenbildwandlerkontrolle durch die Haut bis in den Knochen hinein geführt. Ist die Zielposition erreicht, so wird der Obturator herausgezogen. Das Kanülenrohr stellt nun den Arbeitskanal zum Zielort dar. Durch die Kanüle kann der weiche Ballon bis zur Therapieposition vorgeschoben werden, ohne daß die Gefahr besteht, daß er während des Vorschubes an den Knochenbälgchen hängen bleibt. (Fig. 1a oben zeigt die über Kanüle und Ballon geschobene Einführhilfe.) Ist der Ballon In der Zielposition angelangt, so wird die Einführhilfe soweit zurückgezogen, daß der Ballon durch Injektion von Knochenzement entfaltet werden kann.

Füllmaterial und Ballonmaterial sind so ausgewählt, daß bis zur Aushärtung des Füllmaterials 2-3 % des Materials in die Cavität austreten. Dadurch kommt es zur sicheren Fixierung des Implantats In der Cavität, ohne daß die Gefahr besteht, daß das Füllmaterial in das posterlore Viertel des Wirbel körpers gelangt und dort venöse Gefäße verfüllt (oder mit der Gefahr von Embolien und Infarkten durch frei flottierendes, ausgehärtetes Füllmaterial).

Abhängig von der Ausbildung der Cavität kann auch die Einbindung mehrerer Implantate, ggf. von unterschiedlichen Zugängen her, zweckmäßig sein.

## Patentansprüche

1. Dilatierbares Ballonimplantat zur Einbringung in einen Wirbelkörper und dortigen Verfüllung mit einem aushärtenden Füllmaterial, wobei das Implantat begrenzt flüssigkeitsdurchlässig ausgebildet ist,
**dadurch gekennzeichnet,**
**dass** der Ballon (3) aus einem Material besteht, das an sich flüssigkeitsundurchlässig ist und mit Poren versehen ist, wobei die Poren bis zu 5 %, bezogen auf die Ballonoberfläche, ausmachen.

2. Implantat gemäß Anspruch 1, **dadurch gekennzeichnet, daß** das Material punktiertes Latex ist.

3. Implantat gemäß einem der vorstehenden Ansprüche, **dadurch gekennzeichnet, daß** die Durchlässigkeit des für den Ballon (3) verwendeten Materials für medizinisches Knochenfüllmaterial 0,05 bis 6, vorzugsweise 1 bis 4 % und besonders bevorzugt 2 bis 3 % beträgt.

4. Implantat gemäß einem der vorstehenden Ansprüche, **dadurch gekennzeichnet, daß** das Ballonimplantat (3) zur Einnahme einer vorbestimmten Form im dilatierten Zustand vorgeformt ist.

5. Implantat gemäß einem der vorstehenden Ansprüche, **dadurch gekennzeichnet, daß** es mit einem Verbindungsmittel zur lösbaren und leitenden Verbindung mit einer Einführhülse (2) ausgerüstet ist.

6. Implantat gemäß einem der vorstehenden Ansprüche, **dadurch gekennzeichnet, daß** es am proximalen, offenen Ende eine Materialschwächung als Sollbruchstelle aufweist.

7. Implantat gemäß einem der vorstehenden Ansprüche, **dadurch gekennzeichnet, daß** es am proximalen, offenen Ende mit einem Mittel zum Verschluss des Ballonlumens ausgerüstet ist.

8. Vertebroplastie-Vorrichtung mit einem Ballonimplantat (3) nach einem der vorstehenden Ansprüche, welches mit dem distalen Ende einer Einführhülse (2) verbunden ist, so daß sein Inneres mit dem Einführhülsenlumen kommuniziert.

9. Vorrichtung gemäß Anspruch 8, **dadurch gekennzeichnet, daß** das Implantat (3) mit der Einführhülse (2) durch formschlüssige Verbindung, Verkleben, Verklemmen, Spreizverbindung oder Verschraubung verbunden ist.

10. Vorrichtung gemäß einem der Ansprüche 8 oder 9, **dadurch gekennzeichnet, daß** die Verbindung zwischen Einführhülse (2) und Ballonimplantat (3) lösbar ist.

11. Vorrichtung gemäß einem der Ansprüche 8 oder 9, **dadurch gekennzeichnet, daß** die Verbindung zwischen Einführhülse (2) und Ballonimplantat (3) unlösbar ist, und das Ballonimplantat (3) mit einer Sollbruchstelle ausgestattet ist.

12. Vorrichtung gemäß einem der Ansprüche 8 bis 11, **dadurch gekennzeichnet, daß** das Ballonimplantat (3) mit seinem offenen Ende im inneren Umfang der Einführhülse (2) befestigt ist.

13. Vorrichtung gemäß einem der Ansprüche 8 bis 12, **dadurch kennzeichnet, daß** das Ballonimplantat (3) mit seinem offenen Ende über das distale Einführhülsenende gestülpt und mit dem äußeren Einführhülsenumfang verbunden ist.

14. Vorrichtung gemäß einem der Ansprüche 8 bis 13, **dadurch gekennzeichnet, daß** die Einführhülse (2) am distalen Ende ein zusätzliches Sicherungsmittel (4) für die Befestigung des Ballonimplantats (3) aufweist.

15. Vorrichtung gemäß einem der Ansprüche 8 bis 14, **dadurch gekennzeichnet, daß** die Einführhülse (2) aus einem medizinischen Stahl besteht.

## Claims

1. Dilatable balloon implant, to be placed in a vertebra and to be filled therein with a hardening filler material, the implant being designed to provide limited liquid permeability, **characterized in that** the balloon (3) consists of a material that is inherently liquid impermeable and is provided with pores, the pores accounting for up to 5 % of the balloon surface.

2. Implant according to claim 1, **characterized in that** the material is stippled latex.

3. Implant according to any of the above claims, **characterized in that** the material used for the balloon (3) has a permeability level to medical bone filler material of 0.05 to 6, preferably 1 to 4 and especially preferably 2 to 3%.

4. Implant according to any of the above claims, **characterized in that** the balloon implant (3) is preshaped in such a manner that it assumes a predetermined shape when dilated.

5. Implant according to any of the above claims, **characterized in that** it is provided with a means for joining it, in a separable and conductive manner, to an introduction sleeve (2).

6. Implant according to any of the above claims, **characterized in that** it is provided, at its proximal, open end, with a thinner wall area serving as a predetermined breaking point.

7. Implant according to any of the above claims, **characterized in that** it is provided, at its proximal, open end, with a means for closing the balloon lumen.

8. Vertebroplasty device equipped with a balloon implant (3) according to any of the above claims, which is connected to the distal end of an introduction sleeve (2) in such a manner that the interior of the implant communicates with the introduction sleeve lumen.

9. Device according to claim 8, **characterized in that** the implant (3) is joined to the introduction sleeve (2) by means of a form-fit joining technique, such as gluing, clamping, expanding or bolting.

10. Device according to any of claims 8 or 9, **characterized in that** the connection between the introduction sleeve (2) and the balloon implant (3) is separable.

11. Device according to any of claims 8 or 9, **characterized in that** the connection between the introduction sleeve (2) and the balloon implant (3) is inseparable and that the balloon implant (3) is provided with a predetermined breaking point.

12. Device according to any of claims 8 through 11, **characterized in that** the open end of the balloon implant (3) is attached to the inner circumference of the introduction sleeve (2).

13. Device according to any of claims 8 through 12, **characterized in that** the open end of the balloon implant (3) is pulled over the distal end of the introduction sleeve and attached to the outer circumference of the introduction sleeve.

14. Device according to any of claims 8 through 13, **characterized in that** the introduction sleeve (2) is provided, at its distal end, with an additional means (4) for securing the balloon implant (3) in place.

15. Device according to any of claims 8 through 14, **characterized in that** the introduction sleeve (2) is made of medical grade steel.

## Revendications

1. Implant de type ballon gonflable, destiné à être introduit dans un corps de vertèbres et à y être rempli d'un matériau de remplissage durcissant, l'implant étant réalisé perméable aux liquides de façon limitée, **caractérisé en ce que** le ballon **(3)** est constitué d'un matériau qui est en soi imperméable aux liquides et qui est muni de pores, les pores représentant jusqu'à 5 % de la surface du ballon.

2. Implant selon la revendication 1, **caractérisé en ce que** le matériau est du latex perforé.

3. Implant selon l'une des revendications précédentes, **caractérisé en ce que** la perméabilité du matériau employé pour le ballon **(3)** à un matériau médical de remplissage d'os vaut 0,05 à 6, de préférence 1 à 4 % et de plus grande préférence de 2 à 3 %.

4. Implant selon l'une des revendications précédentes, **caractérisé en ce que** l'implant **(3)** de type ballon est préformé pour prendre une forme prédéterminée une fois gonflé.

5. Implant selon l'une des revendications précédentes, **caractérisé en ce qu'**il est muni d'un moyen de jonction pour établir une jonction amovible et conductrice avec une douille d'introduction **(2).**

6. Implant selon l'une des revendications précédentes, **caractérisé en ce qu'**il présente à l'extrémité proximale ouverte un affaiblissement du matériau servant de zone de rupture imposée.

7. Implant selon l'une des revendications précédentes, **caractérisé en ce qu'**il est muni à l'extrémité proximale ouverte d'un moyen de fermeture de l'ouverture du ballon.

8. Dispositif de vertébroplastie avec un implant **(3)** de type ballon selon l'une des revendications précédentes, qui est relié à l'extrémité distale d'une douille d'introduction **(2),** de sorte que son intérieur communique avec l'ouverture de la douille d'introduction.

9. Dispositif selon la revendication 8, **caractérisé en ce que** l'implant **(3)** est relié à la douille d'introduction **(2)** par une liaison par engagement positif, par collage, par coincement, par écartement ou par vissage.

10. Dispositif selon l'une des revendications 8 ou 9, **caractérisé en ce que** la liaison entre la douille d'introduction **(2)** et l'implant de type ballon **(3)** est amovible.

11. Dispositif selon l'une des revendications 8 ou 9, **caractérisé en ce que** la liaison entre la douille d'introduction **(2)** et l'implant **(3)** de type ballon est inamovible et l'implant **(3)** de type ballon est muni d'une zone de rupture imposée.

12. Dispositif selon l'une des revendications 8 à 11, **caractérisé en ce que** l'implant **(3)** de type ballon est fixé avec son extrémité ouverte dans la circonférence intérieure de la douille d'introduction **(2).**

13. Dispositif selon l'une des revendications 8 à 12, **caractérisé en ce que** l'implant **(3)** de type ballon est rabattu avec son extrémité ouverte sur l'extrémité distale de la douille d'introduction et est relié à la circonférence extérieure de la douille d'introduction.

14. Dispositif selon l'une des revendications 8 à 13, **caractérisé en ce que** la douille d'introduction **(2)** présente à l'extrémité distale un moyen de fixation supplémentaire **(4)** pour la fixation de l'implant **(3)** de type ballon.

15. Dispositif selon l'une des revendications 8 à 14, **caractérisé en ce que** la douille d'introduction **(2)** est composée d'un acier médical.
